# EUROPEAN PATENT SPECIFICATION

(11) **EP 1 731 030 B1**
(45) Date of publication and mention of the grant of the patent: **21.06.2017**
(21) Application number: 05720067.7
(22) Date of filing: 04.03.2005
(51) Int. Cl.: C12N 5/09, A61K 49/00, A01K 67/027, G01N 33/15

(54) **METHOD OF CONSTRUCTING ANIMAL HAVING CANCER CELLS TRANSPLANTED THEREINTO**
VERFAHREN ZUR HERSTELLUNG EINES TIERS MIT HINEINTRANSPLANTIERTEN KREBSZELLEN
METHODE DE CONSTRUCTION D'ANIMAL AYANT DES CELLULES CANCEREUSES TRANSPLANTEES

(30) Priority: 04.03.2004 JP 2004106999
(43) Date of publication of application: 13.12.2006
(73) Proprietor: CellSeed Inc., Tokyo 135-0064 (JP)
(72) Inventor: OKANO, Teruo, Ichikawa-shi, Chiba 272-0827 (JP); KIKUCHI, Akihiko, Tokyo 177-0051 (JP); YAMATO, Masayuki, Tokyo 158-0097 (JP); MASUDA, Akira, Tokyo 136-0072 (JP)
(74) Representative: Hoffmann Eitle
(86) International application number: PCT/JP2005/003795
(87) International publication number: WO 2005/084429

(56) References cited:
- WO-A-91/01760
- JP-A- 5 192 138
- HIROSE M, KWON OH, YAMATO M, KIKUCHI A, OKANO T.: "Creation of designed shape cell sheets that are noninvasively harvested and moved onto another surface" BIOMACROMOLECULES., vol. 1, no. 3, 2000, pages 377-381, XP002544976
- NIESEL DW. ET AL: 'Quantitation of HeLa cell monolayer invasion by Shigella and Salmonella species.' MATERIALS AND METHODS. vol. 22, no. 3, December 1985, pages 897 - 902, XP002989166
- NISHIDA K ET AL: "Functional bioengineered corneal epithelial sheet grafts from sorneal stem cells expanded ex vivo on a temperature-responsive cell culture surface", TRANSPLANTATION, WILLIAMS AND WILKINS, GB, vol. 77, no. 3, 1 February 2004 (2004-02-01), pages 379-385, XP002988152, ISSN: 0041-1337, DOI: 10.1097/01.TP.0000110320.45678.30
- SHIMIZU T ET AL: "Cell sheet engineering for myocardial tissue reconstruction", BIOMATERIALS, ELSEVIER SCIENCE PUBLISHERS BV., BARKING, GB, vol. 24, no. 13, 1 June 2003 (2003-06-01), pages 2309-2316, XP004420022, ISSN: 0142-9612, DOI: 10.1016/S0142-9612(03)00110-8
- KUSHIDA A ET AL: "Two-dimensional manipulation of differentiated Madin-Darby canine kidney (MDCK) cell sheets : the noninvasive harvest from temperature-responsive culture dishes and transfer to other surfaces", JOURNAL OF BIOMEDICAL MATERIALS RESEARCH, WILEY, NEW YORK, NY, US, vol. 54, no. 1, 1 January 2001 (2001-01-01), pages 37-46, XP002903780, ISSN: 0021-9304, DOI: 10.1002/1097-4636(200101)54:1<37::AID-JBM5 >3.0.CO;2-7
- NISHIDA K ET AL: "CORNEAL RECONSTRUCTION WITH TISSUE-ENGINEERED CELL SHEETS COMPOSED OF AUTOLOGOUS ORAL MUCOSAL EPITHELIUM", NEW ENGLAND JOURNAL OF MEDICINE, MASSACHUSETTS MEDICAL SOCIETY, US, vol. 351, no. 12, 16 September 2004 (2004-09-16), pages 1187-1196, XP008049467, ISSN: 0028-4793, DOI: 10.1056/NEJMOA040455
- SUZUKI R. , ARUGA A., KOBAYASHI H., YAMATO M., YAMAMOTO M..: "Development of a novel in vivo cancer model using cell sheet engineering.", ANTICANCER RES., vol. 34, no. 9, 16 September 2014 (2014-09-16), pages 4747-4754, XP055173510,
- AKIMOTO J1, TAKAGI S, NAKAYAMA M, ARAUCHI A, YAMATO M, OKANO T.: "Transplantation of cancerous cell sheets effectively generates tumour-bearing model mice.", J TISSUE ENG REGEN MED.2013 NOV 6., 6 November 2013 (2013-11-06), XP055173503, & AKIMOTO J1, TAKAGI S, NAKAYAMA M, ARAUCHI A, YAMATO M, OKANO T.: , 1 January 2013 (2013-01-01), XP055173503, Retrieved from the Internet: URL:http://onlinelibrary.wiley.com/doi/10. 1002/term.1850/epdf?systemMessage=Wiley+On line+Library+will+be+disrupted+on+7th+Marc h+from+10%3A00-13%3A00+GMT+%2805%3A00-08%3 A00+EST%29+for+essential+maintenance.++Apo logies+for+the+inconvenience. [retrieved on 2015-03-03]

## Description

### TECHNICAL FIELD

This invention relates to a process for preparing cancer cells transplanted non human animals in fields such as biology and medicine.

### BACKGROUND ART

Cancer is the most common cause of death in Japan and it is said that about 30% of Japanese people die of cancer. In spite of the recent development of tailor-made medicine based on genomic information, therapeutics effective against cancer are yet to be discovered. Essential to the development of anti-cancer agents are appropriate cancer-bearing animals and their development is currently in need.

Cancer cells transplanted animals include knockout mice deprived of antioncogenes such as APC and p53, as well as animals in which cancer has been developed by various methods such as the use of chemicals and other carcinogenic agents and direct transplantation of cancer cells of interest. Among these animals, antioncogene knockout mice can be prepared in a fairly short period of time but, on the other hand, they are not easy to adopt since they are fairly expensive and subject to various limitations of organizations entrusted for commissioned production. Cancer development with chemicals requires a prolonged time to develop cancer, so much time is spent before a certain conclusion is reached.

Transplanting cancer cells has the advantage of giving experimental results in a short period of time. On the other hand, the transplanted cancer cells have poor "take" and the size and weight of the transplanted cancer tissue vary so greatly from one animal to another that evaluation of various anti-cancer agents involves difficulty in revealing any significant differences in their efficacy. Reasons for this defect include the poor "take" of the transplanted cancer cells and the leakage of the cancer cells suspension from the site of transplantation; it has therefore been necessary to improve the functions of the cells to be transplanted.

JP 05-192138 A describes a method of skin cells cultivation comprising the steps of preparing a cell culture support which has a surface of its base coated with a polymer having an upper or lower critical temperature for dissolution in water in a range of 0-80°C, cultivating skin cells on the cell culture support at a temperature not higher than the upper critical temperature for dissolution or at a temperature not lower than the lower critical temperature for dissolution, and thereafter adjusting the temperature to above the upper critical temperature for dissolution or below the lower critical temperature for dissolution, whereby the cultured skin cells are detached. This method depends on temperature adjustment for detaching the cells from the culture base coated with the temperature-responsive polymer; however, JP 05-192138 A neither describes nor suggests a method of preparing cancer cells transplanted animals using the thus obtained cells. WO 91/01760 A addresses the problem of providing a non human animal model of cancer comprising transplanted cancer cells and discloses a process for preparing a cancer cell-transplanted non-human animal comprising implanted preparing a cell culture on a biocompatible support, then cultivating cells on the support and transplanting the cancer cells maintained on the (preferably biodegradable) support including a biological response modifier, such as an angiogenic growth factor, to a specified site of a non-human animal on which transplantation is to be performed, for example as a stripe shape.

### DISCLOSURE OF THE INVENTION

### PROBLEMS TO BE SOLVED BY THE INVENTION

The present invention has been accomplished with a view to solving the aforementioned problems of the prior art. Thus, the present invention has as its object providing a new process for preparing cancer cells transplanted non human animals from an entirely different perspective than in the prior art.

### MEANS FOR SOLVING THE PROBLEMS

In order to solve the aforementioned problems, the present inventors made R&D efforts based on a review from various viewpoints. They first prepared a cell culture support coated on a surface with a polymer the hydration force of which would change in a temperature range of 0-80°C; cancer cells were then cultivated on the support in a temperature region where the polymer had weak hydration force; thereafter, the culture solution was adjusted to a temperature at which the polymer had a stronger hydration force, whereby the cultured cancer cells were detached; the detached cancer cells were then transplanted to a specified site of an animal to be treated; surprisingly enough, this method turned out to be an efficient way of cancer cells transplantation. It was also found that the size and/or shape of the cancer tissue in the animal could be controlled by preparing a sheet of the cancer cells in a specified shape of a specified size. The present invention has been accomplished on the basis of these findings. The invention in its broadest sense is as defined in the independent claims.

In general, the present invention provides a process for preparing a cancer cells transplanted animal comprising the steps of preparing a cell culture support coated on a surface with a polymer the hydration force of which will change in a temperature range of 0-80°C, then cultivating cancer cells on the support in a temperature region where the polymer has weak hydration force, thereafter adjusting the culture solution to a temperature at which the polymer has a stronger hydration force, whereby the cultured cancer cells are detached, and transplanting the detached cancer cells to a specified site of a non-human animal on which transplantation is to be performed.

In a preferred embodiment of the process, a cancer cell sheet to be transplanted is prepared in a specified shape of a specified size so that the size and/or shape of the cancer tissue in the animal is controlled.

The present invention also provides a cancer cells transplanted non human animal prepared by the process described above.

The present invention further provides a method of selecting an anti-tumor agent comprising the steps of administering a test substance to an animal before and/or after transplanting cancer cells in the preparation process described above and evaluating the effect of the administered test substance on tumor formation.

### ADVANTAGES OF THE INVENTION

In the process for preparing cancer cells transplanted non human animals described herein, the cultured cancer cells are detached without any enzyme treatment, so the adherent protein remains intact to allow for good "take" after transplantation; as a further advantage, if a sheet of the cancer cells is prepared and applied to an animal, the leakage of the cancer cells suspension that would otherwise occur from the site of transplantation can be prevented to enable efficient preparation of a cancer cells transplanted animal.

### BRIEF DESCRIPTION OF THE DRAWINGS

[Fig. 1] Fig. 1 is a graph showing how the tumor volume in nude mice that were transplanted with a sheet of cancer cells in their back in Example 1 changed over time, with the dimensions of a tumor being measured and its volume calculated for an ellipsoid;
[Fig. 2] Fig. 2 is a graph showing how the tumor volume in the same nude mice that were transplanted with a sheet of cancer cells in their back changed over time, with the dimensions of a tumor being measured and its volume calculated for a cylindroid;
[Fig. 3] Fig. 3 is a photo showing the back of a nude mouse before cancer cells transplantation;
[Fig. 4] Fig. 4 is a photo showing the back of a nude mouse 4 weeks after transplanting a sheet of cancer cells; and
[Fig. 5] Fig. 5 is a photo showing the back of a nude mouse 4 weeks after transplanting a suspension of cancer cells.

### BEST MODE FOR CARRYING OUT THE INVENTION

The cells to be used in the present invention may be any cancer cells that are directly collected from a living tissue; alternatively, they include, but are not limited to, cell lines such as HBC-4, BSY-1, HBC-5, MCF-5. MCF-7. MDA-MB-231, U251. SF-268, SF-295, SF-539, SNB-75, SNB-78, HCC2998, KM-12, HT-29, WiDr, HCT-15, HCT-116, NCI-H23. NCI-H226, NIC-H522, NCI-H460. A549, DMS273, DMS114, LOX-IMVI, OVCAR-3, OVCAR-4, OVCAR-5, OVCAR-8, SK-OV-3, RXF-631L, ACHN, St-4, MKN1, MKN7, MKN28, MKN45, and MKN74. The cells to be used in the invention can be derived from various sources that include but are by no means limited to human being, dog, cat, rabbit, rat, swine and sheep. The culture medium for cultivating cells in the present invention is not limited in any particular way as long as it is conventionally used with the cells to be cultivated.

In the present invention, the cells described above are cultivated on a cell culture support coated on a surface with a polymer the hydration force of which changes in a temperature range of 0-80°C, the cultivation temperature being in a region where the polymer has weak hydration force. The temperature region where the polymer has week hydration force is by no means limited as long as the polymer coating is in a desolvated state and the applicable temperature is typically in the cell cultivating temperature range of 35-38°C, with 37°C being particularly preferred. Cells will adhere and grow if the cell culture support is coated with the polymer in the amount to be described below and if the polymer remains dehydrated. The polymer to be used in the present invention is of such a nature that its hydration force changes abruptly at a temperature characteristic of it in the range of 0-80°C. To be more specific, the polymer makes a sudden shift from a dehydrated to a hydrated state at that characteristic temperature. Being coated with this polymer, the surface of the cell culture support material on which the cells have adhered and grown changes to a non-adherent state, making it possible to detach the cultured cells. According to the present invention, there is no need to use enzymes such as trypsin but one may simply change the cultivation temperature to detach the cultured cells; hence, the detached cell sheet is only lightly damaged, free from the damage it would receive if it were treated with an enzyme such as trypsin. Since the detachment of the cultured cancer cells involves no enzyme treatment, the adherent protein remains intact, assuring good "take" after transplantation; if the cancer cells are in a sheet form, there is another advantage in that the leakage of a cell suspension from the site of transplantation is effectively suppressed to allow for efficient preparation of a cancer cells transplanted animal.

The temperature-responsive polymer to be used in the present invention may be a homo- or copolymer. Examples of such polymer are described in JP 2-211865 A. Specifically, the intended polymer can be obtained by homo- or copolymerization of the monomers listed below. Applicable monomers include (meth)acrylamide compounds, N- (or N,N-di)alkyl-substituted (meth)acrylamide derivatives, and vinyl ether derivatives. In the case of copolymers, any two or more of these monomers may be employed. If desired, they may be copolymerized with other monomers, or the resulting polymers may be subjected to graft polymerization or copolymerization, or mixtures of polymers and copolymers may be employed. The polymers can also be crosslinked to the extent that will not impair their inherent properties. The method of coating the base surface with the various polymers mentioned above is not limited in any particular way and an exemplary method that can be followed is described in JP 2-211865 A. To be specific, coating can be done by subjecting the base and the monomers or polymers mentioned above to irradiation with electron beams (EB), γ-rays or ultraviolet light, or plasma or corona treatment, or organic polymerization reaction; alternatively, physical adsorption can be effected by coating or blending. The amount in which hydrophilic polymers are to be immobilized at the site of cell adhesion is not limited to any particular value as long as they are sufficient to adhere the cells that need to be moved; however, since cancer cells are used in the present invention, the hydrophilic polymers are typically immobilized in an amount of at least 0.4 µg/cm², preferably at least 0.8 µg/cm², and more preferably at least 1.2 µg/cm². The amount of polymer immobilization may be measured by the usual method; in one example, the site of cell adhesion is directly measured by FT-IR-ATR, and in another example, a preliminarily labeled polymer is first immobilized at the site of cell adhesion by the same method and the amount of interest is estimated from the amount of the immobilized labeled polymer. Either of these methods is practically feasible.

The shape of the culture base to be used in the present invention is not limited in any particular way; examples include shapes such as a dish, a multi-well plate, a flask and a cell insert, as well as a flat membrane. The base to be coated with the polymer is one that is customarily used in cell culture and may be exemplified by glass, modified glass, compounds such as polystyrene and poly(methyl methacrylate), and all those materials that generally can be given a shape, including high-molecular weight compounds and ceramics other than those mentioned above.

The temperature-responsive polymer with which the base of the cell culture support is coated for use in the present invention tends to undergo hydration or dehydration in response to a change in temperature within a certain region, which has turned out to range from 0°C to 80°C, preferably from 10°C to 50°C, and more preferably from 20°C to 45°C. Temperatures beyond 80°C are not preferred since cancer cells are prone to die. Temperatures lower than 0°C are also not preferred since the cell growth rate drops considerably or the cancer cells die.

In order to detach and recover the cultured cancer cells from the support material in the present invention, they are optionally brought into intimate contact with a carrier and then the support material to which the cells adhering is adjusted to a temperature at which the polymer coat on the support base undergoes hydration, whereupon the cancer cells can be detached intact from the support together with the carrier. In this case, a water stream may be applied between the cell sheet and the support assure smooth detachment. The detachment of the cell sheet may be effected within the culture solution in which the cells have been cultivated or in other isotonic solutions, whichever is suitable for a specific purpose.

The cancer cells cultivated in the present invention are free from the damage which they would sustain if they were treated with proteolytic enzymes represented by dispase and trypsin. Therefore, the cancer cells detached from the base have an adherent protein and if they are detached in a sheet form, the desmosome structure between cells will be retained to some extent. As a result, the detached cancer cells can adhere satisfactorily to the diseased tissue to which they have been transplanted, thus assuring efficient transplantation. It is generally known that cells treated with the proteolytic enzyme dispase can be detached while retaining 10-60% of the desmosome structure between cells but, on the other hand, most of the basement membrane-like protein between cell and the base is destroyed, with the detached cell sheet having only low strength. In contrast, the cancer cells sheet prepared in the present invention keeps both the desmosome structure and the basement membrane-like protein intact in a respective amount of at least 80%, thereby providing the various advantages already described above.

The foregoing description is paraphrased below by referring to poly(N-isopropylacrylamide) as an example of the temperature-responsive polymer. Poly(N-isopropylacrylamide) is known as a polymer having a lower critical temperature for dissolution at 31°C and if it is in a free state, it undergoes dehydration in water at a temperature above 31°C, with the polymer chains agglomerating to cause turbidity. Conversely, at 31°C and below, the polymer chains become hydrated and dissolve in water. In the present invention, a coat of this polymer is immobilized on a surface of the base such as a Petri dish. Therefore, at a temperature above 31°C, the polymer on the base surface likewise undergoes dehydration but with the polymer chains being immobilized to form a coat on the base surface, the latter comes to show hydrophobicity; on the other hand, at 31°C and below, the polymer on the base surface undergoes hydration but with the coat of polymer chains being immobilized on the base surface, the latter comes to show hydrophilicity. The hydrophobic surface is appropriate for the adhesion and growth of cells whereas the hydrophilic surface hates cell adhesion so much that the cells being cultivated or the cell sheet needs only to be cooled to become detached.

The carrier to be used for bringing the caner cells or the sheet of cancer cells into intimate contact with the culture base is a structure for holding the cells used in the present invention and examples that can be used include high-molecular weight membranes, structures shaped from high-molecular weight membranes, and metallic jigs. If a high-molecular weight membrane is to be used as a material for the carrier, specific examples include poly(vinylidene difluoride) [PVDF], polypropylene, polyethylene, cellulose or its derivatives, paper and like products, chitin, chitosan, collagen, and polyurethane. The shape of the carrier is not limited in any particular way.

The cancer cells to be transplanted in the present invention have good "take", so they only need to be transplanted in a total number of 1 x 10⁵ cells or less, preferably 5 x 10⁵ cells or less, and more preferably 8 x 10⁵ or less. In the case of the present invention, transplanting more than 8 x 10⁵ cancer cells is advantageous since a large enough cancer tissue can be obtained but, on the other hand, an undesirably large number of cells have to be handled at a time. The site of transplantation is not limited at all and cancer cells may be transplanted under the skin or they may be directly transplanted to the tissue derived from specific cancer cells.

Animals that can be used as recipients for transplantation in the present invention include, but are not limited to, nude mouse, rat, mouse, guinea pig, and rabbit.

As described above, the high-take cancer cells to be used in the present invention can adhere very satisfactorily to living tissues to ensure the preparation of cancer cells transplanted animals within a very short period of time that has been quite impossible to realize in the prior art.

The cancer cells transplanted animal prepared in the present invention can be employed in a method of selecting an anti-tumor agent comprises the steps of administering a test substance to an animal before and/or after transplanting cancer cells in the preparation process and evaluating the effect of the administered test substance on tumor formation.

### Examples

The present invention is described below in greater detail with reference to examples which are by no means intended to limit the scope of the present invention.

### Example 1

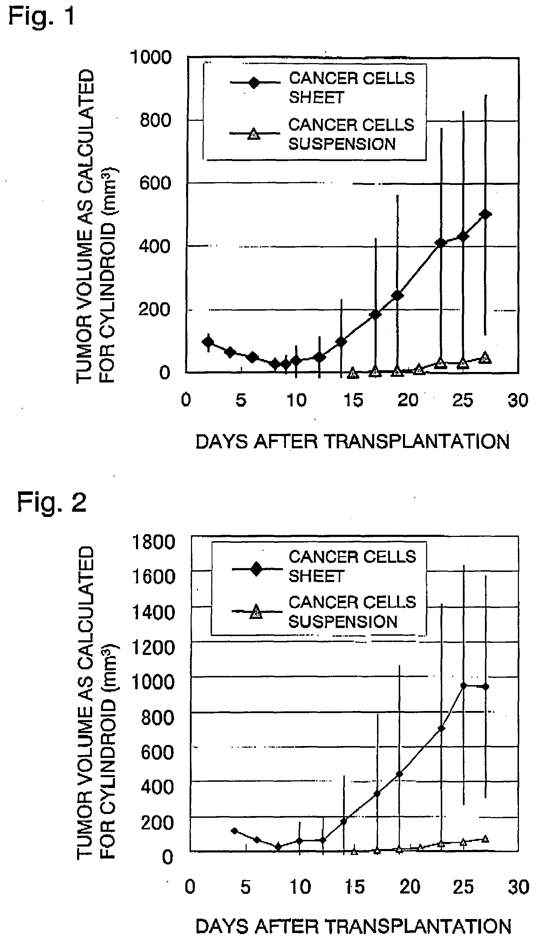

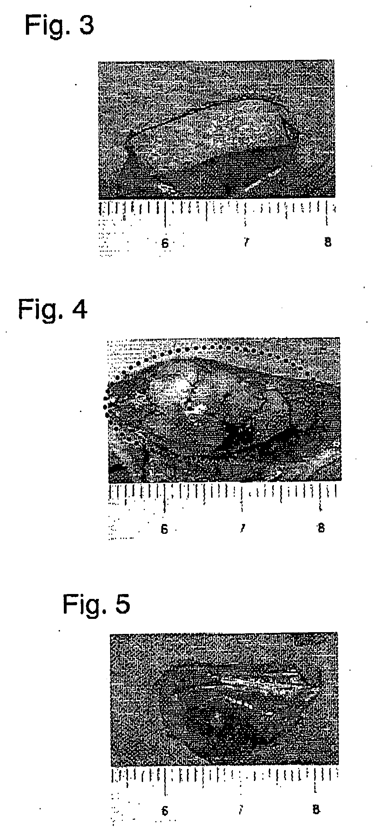

A cell culture base was coated with the temperature-responsive polymer poly(N-isopropylacrylamide) in an amount of 2.0 µg/cm² and the cancer cells NCI-H460 was cultivated (2 x 10⁴ cells were seeded; 37°C in 5% CO₂). Three days later, the cancer cells (NCI-H460) on the culture base were confirmed to have become confluent; thereafter, a cultured cell moving jig comprising a polyacrylic plate coated with a fibrin gel was gently placed over the cultured cell sheet so that the cultured cancer cells adhered to it; then, the cell culture base was cooled at 20°C for 60 minutes. After the cooling, the detached cell sheet was collected from the jig together with the fibrin gel and a piece of the gel with the adhering cell sheet (7 mm x 17 mm x 2 mm; 5 x 10⁵ cells) was transplanted subcutaneously to the back of each of 10 nude mice. The dimensions of the tumor that developed after the transplantation were measured over the skin with a micrometer and the results obtained by calculating the volume of the tumor assuming that it was an ellipsoid are shown in Fig. 1 (volume of ellipsoid = π/6 x major axis of the tumor x minor axis of the tumor x thickness of the tumour). The results obtained by calculating the volume of the tumor assuming that it was a cylindroid are shown in Fig. 2 (volume of cylindroid = π/4 x major axis of the tumor x minor axis of the tumor x thickness of the tumor). Four weeks after the transplantation, the mean volume of ellipsoid was 581.7 ± 566.3 mm³, the mean volume of cylindroid was 1302.7 ± 1007.9 mm³, and the mean tumor weight was 776.9 ± 534 mg. Fig. 3 is a photo showing the back of a nude mouse before the transplantation and Fig. 4 is a photo showing the back of the same nude mouse 4 weeks after transplanting the cancer cells sheet.

### Comparative Example 1

The cancer cells NCI-H460 was cultivated on a cell culture base with no temperature-responsive polymer coat on its surface (2 x 10⁴ cells were seeded; 37°C in 5% CO₂). Three days later, the cancer cells (NCI-H460) on the culture base were confirmed to have become confluent; thereafter, trypsin treatment was performed to recover the cancer cells. A suspension of the recovered cancer cells (5 x 10⁵) was transplanted subcutaneously to the back of each of two nude mice. As in Example 1, the tumor volume was calculated on the assumption that it was either an ellipsoid or a cylindroid, and the respective results are also shown in Figs. 1 and 2. Four weeks after the transplantation, the mean volume of ellipsoid was 40.7 mm³, the mean volume of cylindroid was 60.7 mm³, and the mean tumor weight was 74.2 mg. Fig. 5 is a photo showing the back of a nude mouse 4 weeks after transplanting the suspension of cancer cells.

### Example 2

A cell culture base was coated with the temperature-responsive polymer poly(N-isopropylacrylamide) in an amount of 1.9 µg/cm² and the cancer cells A-549 was cultivated (2 x 10⁴ cells were seeded; 37°C in 5% CO₂). Three days later, the cancer cells (A-549) on the culture base were confirmed to have become confluent; thereafter, a poly(vinylidene difluoride) [PVDF] membrane not coated with a fibrin gel was gently placed over the cultured cell sheet so that the cultured cancer cells adhered to it: then, the cell culture base was cooled at 20°C for 60 minutes. After the cooling, a sheet of cancer cells (7 mm x 17 mm x 2 mm: 5 x 10⁵ cells) was detached together with PVDF membrane. The back of each of 10 nude mice was incised linearly beneath the skin and the subcutaneous tissue was detached with forceps to create a pocket, into which the above-described cancer cells sheet was inserted. After the inserting, the incised part was sutured to complete the transplantation. The dimensions of the tumor that developed after the transplantation were measured over the skin with a micrometer and the volume of the tumor was calculated assuming that it was an ellipsoid. The volume of the tumor was also calculated, this time on the assumption that it was a cylindroid (volume of cylindrold = π/4 x major axis of the tumor x minor axis of the tumor x thickness of the tumor). Four weeks after the transplantation, the mean volume of ellipsoid was 578.7 ± 322.8 mm³, the mean volume of cylindroid was 1258.7 ± 897.9 mm³, and the mean tumor weight was 785.4 ± 394 mg. The decrease in the tumor volume that occurred immediately after the transplantation as shown in Fig. 1 (tumor assumed as an ellipsoid) and Fig. 2 (as a cylindroid) was absent and the tumor grew bigger as more days elapsed after the transplantation.

### Example 3

The cancer cells transplanted animals prepared in Example 2 were administered a total of four times on a once-a-week basis with 3 mg of 5-fluorouracil (5-FU), a known anti-tumor agent, through the tail vein as it was dissolved in 0.3 ml of 1% ethyl alcohol containing physiological saline. Four weeks after the administration, the mean volume of ellipsoid was 279.6 ± 127.1 mm³, the mean volume of cylindroid was 619.3 ± 262.9 mm³, and the mean tumor weight was 369.3 ± 123 mg. As it turned out, the cancer cells transplanted animals described in the present invention got the volume and weight of the tumor to be reduced by receiving the anti-tumor agent. Obviously, the cancer cells transplanted animals of the present invention are useful in selecting an effective anti-tumor agent.

### INDUSTRIAL APPLICABILITY

According to the process of the present invention, cultured cancer cells can be conveniently detached without using any proteolytic enzyme and cancer cells transplanted animals can be prepared efficiently.

## Claims

1. A process for preparing a cancer cell-transplanted non-human animal comprising the steps of preparing a cell culture support coated on a surface with a polymer the hydration force of which changes in a temperature range of 0-80°C, then cultivating cancer cells on the support in a temperature region where the polymer has weak hydration force, thereafter adjusting the culture solution to a temperature at which the polymer has a stronger hydration force, whereby the cultured cancer cells are detached from the cell culture support without being treated with a proteolytic enzyme, and transplanting the detached cancer cells to a specified site of a non-human animal on which transplantation is to be performed, wherein the detached cancer cells are in a sheet form, wherein the polymer the hydration force of which changes in a temperature range of 0-80°C is poly(N-isopropylacrylamide), and wherein the cancer cells are of a transplantable cell line or have been collected from a living tissue.

2. The process for preparing a cancer cell-transplanted non-human animal according to claim 1, wherein the cancer cell sheet to be transplanted is prepared in a specified shape of a specified size so that the size and/or shape of the cancer tissue in the non-human animal is controlled.

3. The process for preparing a cancer cell-transplanted non-human animal according to any one of claims 1-2, wherein a carrier is placed in intimate contact over the cultured cells at the end of cultivation and the cells are detached intact together with the carrier.

4. The process for preparing a cancer cell-transplanted non-human animal according to any one of claims 1-3, the cancer cells are of transplantable cell line selected from the group consisting of HBC-4, BSY-1, HBC-5, MCF-5, MCF-7, MDA-MB-231, U251, SF-268, SF-295, SF-539, SNB-75, SNB-78, HCC2998, KM-12, HT-29, WiDr. HCT-15, HCT-116, NCI-H23, NCI-H226, NIC-H522, NCI-H460, A549, DMS273, DMS114, LOX-IMVI, OVCAR-3, OVCAR-4, OVCAR-5, OVCAR-8, SK-OV-3, RXF-631L, ACHN, St-4, MKN1, MKN7, MKN28, MKN45, and MKN74.

5. The process for preparing a cancer cell-transplanted non-human animal according to any one of claims 1-3, wherein the cancer cells are collected from a living tissue.

6. The process for preparing a cancer cell-transplanted non-human animal according to any one of claims 1-5, wherein no more than 8 x 10⁵ cells are transplanted.

7. The process for preparing a cancer cell-transplanted non-human animal according to any one of claims 1-6, wherein the cancer cells are derived from human being.

8. The process for preparing a cancer cells transplanted non-human animal according to any one of claims 1-7, wherein the model animal for carcinogenesis is a nude mouse, a rat, a mouse, a guinea pig, or a rabbit.

9. A cancer cell-transplanted non-human animal prepared by the process according to any one of claims 1-8.

10. A method of selecting an anti-tumor agent comprising the steps of i) administering a test substance to an animal before and/or after ii) transplanting cancer cells in the process of preparing a cancer cell-transplanted non-human animal by the process according to any one of claims 1-8 and iii) evaluating the effect of the administered test substance on tumor formation.

## Patentansprüche

1. Verfahren zur Herstellung eines nicht-menschlichen Tieres mit transplantierter Krebszelle, umfassend die Schritte des Herstellens eines Zellkulturuntergrunds, der auf einer Oberfläche mit einem Polymer, dessen Hydrationskraft sich in einem Temperaturbereich von 0-80°C ändert, beschichtet ist, dann des Kultivierens von Krebszellen auf dem Untergrund in einer Temperaturzone, wo das Polymer eine schwache Hydrationskraft aufweist, danach des Einstellens der Kulturlösung auf eine Temperatur, bei der das Polymer eine stärkere Hydrationskraft aufweist, wobei die kultivierten Krebszellen vom Zellkulturuntergrund losgelöst werden ohne mit einem proteolytischen Enzym behandelt zu werden, und des Transplantierens der losgelösten Krebszellen an eine festgelegte Stelle eines nicht-menschlichen Tieres, an dem die Transplantation durchgeführt werden soll, wobei die losgelösten Krebszellen in der Form einer Schicht (sheet) vorliegen, wobei das Polymer, dessen Hydrationskraft sich in einem Temperaturbereich von 0-80°C ändert, Poly(N-Isopropylacrylamid) ist, und wobei die Krebszellen von einer transplantierbaren Zelllinie stammen oder aus einem lebenden Gewebe gewonnen wurden.

2. Verfahren zur Herstellung eines nicht-menschlichen Tieres mit transplantierter Krebszelle gemäß Anspruch 1, wobei die zu transplantierende Krebszellschicht in einer festgelegten Form einer festgelegten Größe hergestellt wird, sodass die Größe und/oder Form des Krebsgewebes im nicht-menschlichen Tier kontrolliert wird.

3. Verfahren zur Herstellung eines nicht-menschlichen Tieres mit transplantierter Krebszelle gemäß einem der Ansprüche 1-2, wobei am Ende der Kultivierung ein Träger in engem Kontakt über die kultivierten Zellen gelegt wird und die Zellen intakt zusammen mit dem Träger losgelöst werden.

4. Verfahren zur Herstellung eines nicht-menschlichen Tieres mit transplantierter Krebszelle gemäß einem der Ansprüche 1-3, wobei die Krebszellen von einer transplantierbaren Zelllinie stammen, die ausgewählt ist aus der Gruppe, bestehend aus HBC-4, BSY-1, HBC-5, MCF-5, MCF-7, MDA-MB-231, U251, SF-268, SF-295, SF-539, SNB-75, SNB-78, HCC2998, KM-12, HT-29, WiDr. HCT-15, HCT-116, NCI-H23, NCI-H226, NIC-H522, NCI-H460, A549, DMS273, DMS114, LOX-IMVI, OVCAR-3, OVCAR-4, OVCAR-5, OVCAR-8, SK-OV-3, RXF-631L, ACHN, St-4, MKN1, MKN7, MKN28, MKN45 und MKN74.

5. Verfahren zur Herstellung eines nicht-menschlichen Tieres mit transplantierter Krebszelle gemäß einem der Ansprüche 1-3, wobei die Krebszellen aus einem lebenden Gewebe gewonnen werden.

6. Verfahren zur Herstellung eines nicht-menschlichen Tieres mit transplantierter Krebszelle gemäß einem der Ansprüche 1-5, wobei nicht mehr als 8 x 10⁵ Zellen transplantiert werden.

7. Verfahren zur Herstellung eines nicht-menschlichen Tieres mit transplantierter Krebszelle gemäß einem der Ansprüche 1-6, wobei die Krebszellen von einem Menschen abgeleitet sind.

8. Verfahren zur Herstellung eines nicht-menschlichen Tieres mit transplantierter Krebszelle gemäß einem der Ansprüche 1-7, wobei das Modelltier für die Karzinogenese eine Nacktmaus, eine Ratte, eine Maus, ein Meerschweinchen oder ein Kaninchen ist.

9. Nicht-menschliches Tier mit transplantierter Krebszelle, hergestellt durch das Verfahren gemäß einem der Ansprüche 1-8.

10. Verfahren zum Selektieren eines Antitumor-Mittels, umfassend die Schritte:
i) Verabreichen einer Testsubstanz an ein Tier vor und/oder nach
ii) Transplantieren von Krebszellen im Verfahren zur Herstellung eines nicht-menschlichen Tieres mit transplantierter Krebszelle durch das Verfahren gemäß einem der Ansprüche 1-8 und
iii) Bewerten des Effekts der verabreichten Testsubstanz auf die Tumorbildung.

## Revendications

1. Procédé de préparation d'un animal non humain transplanté avec des cellules cancéreuses, comprenant les étapes consistant à préparer un support de culture cellulaire appliqué sur une surface avec un polymère dont la force d'hydratation change dans une plage de températures de 0-80 °C, puis à mettre des cellules cancéreuses en culture sur le support dans une zone de températures où le polymère possède une faible force d'hydratation, à ajuster par la suite la solution de culture à une température à laquelle le polymère possède une force d'hydratation plus importante, moyennant quoi les cellules cancéreuses en culture se détachent du support de culture cellulaire sans être traitées avec une enzyme protéolytique, et à transplanter les cellules cancéreuses détachées sur un site spécifié d'un animal non humain pour lequel une transplantation doit être réalisée, où les cellules cancéreuses détachées sont sous forme de feuillet, où le polymère dont la force d'hydratation change dans une plage de températures de 0-80 °C est le poly(N-isopropylacrylamide), et où les cellules cancéreuses proviennent d'une lignée cellulaire transplantable ou ont été collectées à partir d'un tissu vivant.

2. Procédé de préparation d'un animal non humain transplanté avec des cellules cancéreuses selon la revendication 1, dans lequel le feuillet de cellules cancéreuses devant être transplanté est préparé selon une forme spécifiée ayant une taille spécifiée de manière à contrôler la taille et/ou la forme du tissu cancéreux chez l'animal non humain.

3. Procédé de préparation d'un animal non humain transplanté avec des cellules cancéreuses selon l'une quelconque des revendications 1 à 2, dans lequel un véhicule est placé en contact étroit sur les cellules en culture à la fin de la culture et les cellules se détachent avec le véhicule sous une forme intacte.

4. Procédé de préparation d'un animal non humain transplanté avec des cellules cancéreuses selon l'une quelconque des revendications 1 à 3, les cellules cancéreuses provenant d'une lignée cellulaire transplantable sélectionnée dans le groupe consistant en HBC-4, BSY-1, HBC-5, MCF-5, MCF-7, MDA-MB-231, U251, SF-268, SF-295, SF-539, SNB-75, SNB-78, HCC2998, KM-12, HT-29, WiDr. HCT-15, HCT-116, NCI-H23, NCI-H226, NIC-H522, NCI-H460, A549, DMS273, DMS114, LOX-IMVI, OVCAR-3, OVCAR-4, OVCAR-5, OVCAR-8, SK-OV-3, RXF-631L, ACHN, St-4, MKN1, MKN7, MKN28, MKN45, et MKN74.

5. Procédé de préparation d'un animal non humain transplanté avec des cellules cancéreuses selon l'une quelconque des revendications 1 à 3, dans lequel les cellules cancéreuses sont collectées à partir d'un tissu vivant.

6. Procédé de préparation d'un animal non humain transplanté avec des cellules cancéreuses selon l'une quelconque des revendications 1 à 5, dans lequel 8 x 10⁵ cellules au maximum sont transplantées.

7. Procédé de préparation d'un animal non humain transplanté avec des cellules cancéreuses selon l'une quelconque des revendications 1 à 6, dans lequel les cellules cancéreuses sont dérivées d'un être humain.

8. Procédé de préparation d'un animal non humain transplanté avec des cellules cancéreuses selon l'une quelconque des revendications 1 à 7, dans lequel le modèle animal de carcinogenèse est une souris nude, un rat, une souris, un cobaye, ou un lapin.

9. Animal non humain transplanté avec des cellules cancéreuses préparé par le procédé selon l'une quelconque des revendications 1 à 8.

10. Procédé de sélection d'un agent antitumoral, comprenant les étapes consistant à i) administrer une substance de test à un animal avant et/ou après ii) une transplantation de cellules cancéreuses selon le procédé de préparation d'un animal non humain transplanté avec des cellules cancéreuses par le procédé selon l'une quelconque des revendications 1 à 8 et iii) évaluer l'effet de la substance de test administrée sur la formation de tumeurs.
